# EUROPEAN PATENT SPECIFICATION

(11) **EP 3 773 244 B1**
(45) Date of publication and mention of the grant of the patent: **24.07.2024**
(21) Application number: 19778317.8
(22) Date of filing: 28.03.2019
(51) Int. Cl.: A61B 17/29, A61B 34/00, A61B 34/20

(54) **CONTROL UNIT FOR A MEDICAL DEVICE**
STEUEREINHEIT FÜR EINE MEDIZINISCHE VORRICHTUNG
UNITÉ DE COMMANDE POUR UN DISPOSITIF MÉDICAL

(30) Priority: 29.03.2018 US 201862649634 P
(43) Date of publication of application: 17.02.2021
(73) Proprietor: Human XTensions Ltd., 4250574 Netanya (IL)
(72) Inventor: SHOLEV, Mordehai, 3783000 Doar-Na Alona (IL); KAUFMAN, Assaf, 7680500 Tal Shahar (IL); ELIHAY, Liran, 8201220 Kiryat Gat (IL)
(74) Representative: Röggla, Harald
(86) International application number: PCT/IL2019/050361
(87) International publication number: WO 2019/186563

(56) References cited:
- WO-A1-2017/031132
- WO-A1-2018/165320
- US-A1- 2008 255 607
- US-A1- 2012 078 243
- US-A1- 2015 173 837
- US-A1- 2016 184 040
- US-A1- 2016 256 155
- US-A1- 2017 157 361

## Description

### FIELD AND BACKGROUND OF THE INVENTION

The present invention relates to a control unit for a medical device such as a minimally invasive surgical tool.

Embodiments of the present invention relate to a control unit that includes a housing and a sensing unit for translating a rotation angle of the housing to a different rotation angle of an articulating region of a steerable device shaft attached to the housing.

Minimally invasive procedures are performed through a small diameter access site in a tissue wall or through a natural orifice. Such procedures minimize trauma to tissue and organs and greatly reduce the patient's recovery period.

In endoscopic procedures performed through a tissue access site (e.g. laparoscopic procedures) a small incision is made in a tissue wall and a small cannula, termed a trocar, is inserted through the incision. The trocar defines a passageway through which various surgical tools (laparoscopes) can be inserted to perform cutting, suturing and removal of tissue.

In endoscopic procedures performed through a natural opening, an endoscope is inserted through the mouth, urethra, anus, etc. and guided to a tissue location in the GI tract, vaginal cavity or bladder to perform a diagnostic or surgical procedure. Endoscopic procedures also include Natural Orifice Transluminal Endoscopic Surgery (NOTES) in which an endoscopic tool is passed through the natural orifice and then through an internal incision in the stomach, vagina, bladder or colon, thus avoiding any external incisions or scars.

Minimally invasive surgical tools are guided within the body using an extracorporeal user control unit which transfers hand/arm movement of the user to movement and actuation (collectively 'operation') of the surgical tool. Thus, the control unit enables the user to control the operation of a surgical tool within the body from outside the body. Many types of tools can be controlled in this manner ranging grasper and scissor-like tools and cameras to complex robotic systems.

Numerous types of surgical tool controllers are known in the art, see for example, US7996110, US7963913, US8521331, US8398541, US8939891, US9050120 US8332072, US20100170519, US20090036901, US20140222023, and US20140228631.
US 2015/0173837 A1 discloses a surgical system which may include a surgical probe having a deflectable tip, and a plurality of guide arms that are movable so as to cause the tip to deflect. Thus, the surgical probe can be steered toward a target anatomical location.

US 2017/0157361 A1 discloses interface devices, systems and methods which can be used for selectively bending of, altering the bend characteristics of, and/or altering the lengths of catheter bodies, guidewires, steerable trocars, and other flexible structures inserted into a patient during use. WO 2018/165320 A1 discloses a system comprising a medical tool including a shaft having proximal and distal ends and an articulatable distal portion coupled to the distal end of the shaft. The system also comprises a processing unit including one or more processors.

US 2016/184040 A1 discloses a control unit for a medical device with a shaft and an end effector and comprising a user interface, sensors, and a drive unit.

Commercially available robotic tool controllers such as the Da Vinci, TransEnterix and Titan systems are large and heavy and force the surgeon to sit in a console away from the patient bed. Such controllers are operated via a hand/finger levers or handles as well as foot pedals and require a high degree of coordination to smoothly operate the robotic surgical tools.

There is thus a need for a control unit that enables a surgeon to intuitively and easily maneuver a surgical tool inside the body using a single hand while allowing precise control and scaling through a wide range of device and effector-end movements.

### SUMMARY OF THE INVENTION

The invention relates to a control unit for a medical device having an articulating shaft and an end effector as defined in claim 1. Further embodiments are specified in the dependent claims. According to one aspect of the present disclosure there is provided a control unit for a medical device having an articulating shaft, the control unit comprising (a) an interface being engageable by a hand of a user, the interface being immovably attached to a housing of the control unit, the housing being attachable to the medical device; and (b) a sensing unit for translating a rotation angle of the housing into a different rotation angle of an articulated region of the shaft.

According to one embodiment of the present invention the rotational angle of the housing is scaled up or down into the different rotation angle of the articulated region of the shaft.

According to one embodiment of the present invention the interface is a palm interface operable via a palm of the hand.

According to one embodiment of the present invention the control unit further comprising a finger interface being pivotally attached to the housing and being engageable by one or more fingers of the hand.

According to one embodiment of the present invention the control unit further comprising a restraint being pivotally attached to the palm interface and having an element capable of elastically deforming to apply a restraining force to a back of a hand when the palm is engaged with the palm interface.

According to one embodiment of the present invention the control unit further comprising a drive unit in the housing.

According to one embodiment of the present invention the finger interface includes levers simultaneously operable via thumb and index finger.

According to one embodiment of the present invention the finger interface is configured for operating articulation of the shaft of the medical device and operating an effector end of the medical device.

According to one embodiment of the present invention the drive unit includes at least one motor for controlling the shaft of the medical device.

According to one embodiment of the present invention the sensing unit includes an inertial measurement unit.

According to one embodiment of the present invention a signal from the inertial measurement unit operates the at least one motor to rotate the shaft.

According to one embodiment of the present invention the at least one motor rotates the articulation region only when in a deflected position.

According to another aspect of the present invention there is provided a system comprising the control unit attached to a minimally invasive medical device having an articulatable shaft.

According to yet another aspect of the present disclosure there is provided a control unit for a medical device, the control unit comprising: (a) an interface for controlling a position and function of an effector end attached to a shaft of the medical device via steerable articulation of the shaft; and (b) a sensing unit for measuring a position of the control unit and the effector end of the medical device and for maintaining an orientation of the effector end with respect to a tissue when the control unit is maneuvered with respect to the tissue.

According to one embodiment of the present invention the interface is engageable by a palm and fingers of a user.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE SEVERAL VIEWS OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1A illustrates one embodiment of the control unit of the present invention attached to a laparoscope.
FIG. 1B illustrates the pitch, roll and yaw axis of the control unit of the present invention.
FIG. 1C illustrates the coordinate systems of the present invention.
FIG. 1D illustrates the relation between the coordinate systems of the present invention.
FIGs. 1E-F illustrates the coordinate system of the shaft articulation of the present invention.
FIG. 1G illustrates the relation between the coordinate systems of the articulation and the control unit the present invention.
FIGs. 2A-B illustrate positioning of the surgeon's hand against the control unit interface.
FIG. 3 is a general view of one embodiment of a motorized laparoscopic tool and attached control unit.
FIG. 4A illustrates the movement of the palm and finger interfaces of one embodiment of the control unit of the present invention.
FIG. 4B illustrates corresponding movements at the articulatable region and effector end of the attached surgical device.
FIG. 5A illustrates in more details the interface of the present invention.
FIGs. 5B-G illustrate control positions of the interface of one embodiment of the control unit of the present invention.
FIGs. 6A-D illustrate one embodiment of the finger interface of one embodiment of the control unit of the present invention.
FIGs. 7A-C illustrate one embodiment of the palm interface of one embodiment of the control unit of the present invention.
FIGs. 8A-F illustrate the mechanical structure and components of the finger and palm interface sensors of one embodiment of the control unit of the present invention.
FIGs. 9A-D illustrate several operational modes of one embodiment of the control unit of the present invention.
FIG. 10 is a flow chart diagram illustrating how signals from the user interface are translated into movement of a laparoscopic tool attached to one embodiment of the control unit of the present invention.
FIG. 11 is a flow chart diagram illustrating how signals from the Inertial measurement unit (IMU) sensor are translated into Pitch, Roll and Yaw angles using the Attitude and heading reference system (AHRS) and are converted to articulation motor commands.
FIG. 12 Illustrates a prototype control unit constructed in accordance with the teachings of the present invention.

### DESCRIPTION OF THE PREFERRED EMBODIMENTS

The present invention relates to a control unit for a medical device and, more particularly, to a control unit and integrated user interface which enable translation of natural hand movements to an attached medical tool such as a laparoscopic tool to thereby enable precise and fine control over the position and function of the medical device.

The principles and operation of the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Control units for surgical tools are well known in the art and are used for controlling mechanical, motorized or robotic tools. Such controllers can be used to accurately position and control surgical instruments within the body, however, they can be bulky and difficult to operate and oftentimes require a long training period to master.

In laparoscopic surgery, a surgeon has to position the distal end of a medical device shaft (carrying an effector end e.g., grasper, cutter, needle holder) adjacent to treated tissue. In order to correctly position the laparoscope, the surgeon has to spatially orient the entire laparoscope, control deflection of an articulatable region of the shaft and actuate the tissue manipulating end.

A surgeon typically uses a manual interface (handle) of a surgical tool for positioning, maneuvering, holding and operating the medical device and effector end at the tissue site of interest. While presently used device interfaces can provide such functionality, they can be limited by a tradeoff between maneuverability and operability of the entire device and its effector end thus requiring considerable time and effort on the part of the surgeon to complete a minimally invasive treatment procedure.

While reducing the present invention to practice, the present inventor set out to design a surgical tool control unit that can be used to easily and naturally control one or more surgical tools while allowing scaling of anatomically-limited hand/arm movements thereby greatly facilitating effector-end positioning at a tissue site of interest.

Previous applications filed by the present inventors disclosed a controller having a palm interface capable of tilting and rotating (yaw of palm) with respect to the housing of the control unit. While experimenting with such a palm interface, the present inventors discovered that the range of motion achieved by a surgeon when rotating a fixed palm interface left and right can be used to scale rotation of an articulated region of an attached surgical tool shaft.

Thus, in order to more effectively control the articulating region, the present inventors devised a control unit that includes a palm interface that is fixedly attached to the control unit housing (and thus does not move with respect thereto) and a sensing unit for scaling left-right rotation of the housing into a scaled (0≤scale) amount of roll of the plane of the articulation of a steerable surgical tool shaft (forming a part of a medical device attached to the housing).

Thus, according to one aspect of the present invention there is provided a control unit for a medical device.

The control unit includes a drive unit and attached user interface. As is further described herein under, the interface is operated by a single hand of a user and actuates motors and mechanical components within the control unit to thereby control positioning, movement and operation of a medical device attached to the control unit.

The user interface controls device positioning, movement and effector end positioning deflection (through shaft articulation and/or effector end tilting) and operation. The user interface includes a palm interface (that can be shaped as a dome) which is fixedly (immovably) mounted on a fixed support attached to a housing of the control unit. The palm interface is engageable by a palm of a hand (by simply resting the palm of the hand on the dome-shaped palm interface) and enables the user to rotate the housing (i.e. to rotate the palm left and right - such rotation is also referred to herein as roll rotation or simply rotation when the control unit and attached device are positioned upright) as well as tilt the entire housing (and attached medical device) with respect to tissue access site.

To maintain the palm of a user against the first interface through the device positioning maneuvers (tilting and rotating as well as up and down movements), the control unit further includes a restraint which is pivotally attached to the palm interface and includes an element that is capable of elastically deforming to apply a restraining force to a back of the hand (dorsum) when the palm is engaged with the palm interface. When this restraint engages the back of the hand, the element elastically deforms and applies a downward force to the back of the hand thus maintaining the hand against the palm interface and enables precise control of this interface, as well as, enabling the user to pull up on the medical device.

The control unit also includes a finger interface which is pivotally attached to the palm interface and is engageable by one or more fingers of the hand.

The control unit of the present invention can be used with any medical device configured for viewing or manipulating tissue at a site of treatment in or on the body of a mammal (e.g. human subject).

The medical device can be a surgical device used in minimally invasive surgery. Such a device typically includes a device body attached to a rigid or steerable shaft. The shaft can carry an effector end that is positioned within a body of a subject through an access site. The shaft and effector end are controlled by a control unit attached to the device body from outside the body (extra corporeally). Examples of medical devices that can be operated by the control unit of the present invention include an endoscope (e.g. laparoscope or thorascope), a catheter, a surgical holder and the like.

The user interface of the present invention is particularly suitable for use with a laparoscopic device having a steerable shaft and a distally-mounted instrument such as a grasper needle holder or cutter.

Laparoscopes are widely used in minimally invasive surgery for viewing or treating organs, cavities, passageways, and tissues. Generally, such devices include an elongated shaft which is designed for delivering and positioning a distally-mounted instrument (effector end) such as a scalpel, a grasper or a camera/camera lens) within a body cavity, vessel or tissue.

Since such devices are delivered though a delivery port positioned through a small incision in a tissue wall (e.g. abdominal wall), and are utilized in an anatomically constrained space (e.g., abdominal cavity), a steerable shaft is advantageous since it can be steered or maneuvered inside the body using controls positioned outside the body (at the proximal end of the medical device). Such steering enables an operator to guide the device within the body and accurately position the distally-mounted instrument at an anatomical landmark.

Numerous examples of steerable devices are known in the art, see for example, U.S. Pat. Nos. 2,498,692; 4,753,223; 6,126,649; 5,873,842; 7,481,793; 6,817,974; 7,682,307 and U.S. Patent Application Publication No. 20090259141.

Deflection of one or more articulating regions of the shaft can be effected via one or more control wires which run along the shaft of the device to the distal end of the steerable portion. The proximal end of each control wire is connected to the motorized mechanism of the control unit; pulling of the wire applies forces that deflect a portion of the shaft with relation to the pulled wire.

The device effector end is controlled via one or more cables which are similarly connected to the control unit and actuated by the user interface.

Thus, the user interface and control unit of a steerable device such as a steerable laparoscope provides three separate functions, positioning of the device shaft with respect to the tissue access site (up/down, tilt, roll), deflection of the steerable portion, and actuation of the distally mounted instrument.

In addition to the above, the present control unit further includes a sensing unit for translating rotation of the control unit housing (and attached medical device) as effected via the palm interface into a scaled rotation of an articulated region of the shaft of an attached medical device.

The user interface of the present invention provides these functions via movement of three separate limb joint and muscle groups.
(i) The shaft of the device is moved up and down, side to side and rotated left/right with respect to the tissue access site by hand and arm movement (primarily about the wrist, elbow and/or shoulder joints). Rotation (as effected via wrist, elbow and/or shoulder movements) is scaled (e.g. increase in rotation angle) by the sensing unit positioned in the housing of the control unit of the present invention.
(ii) Articulation of the shaft is controlled via hand movement (primarily via finger movement combined with the small movement of the wrist joint). This is achieved by tilting the finger interface.
(iii) The distally mounted instrument is actuated via finger movement (primarily about the inter-phalangeal joints and the metacarpal-phalangeal joints).

By scaling the rotation of the housing while the surgeon uses the control unit, the present control unit enables greater and more natural maneuverability-a laparoscope can be operated using less effort and without requiring extreme maneuvering of body and limbs

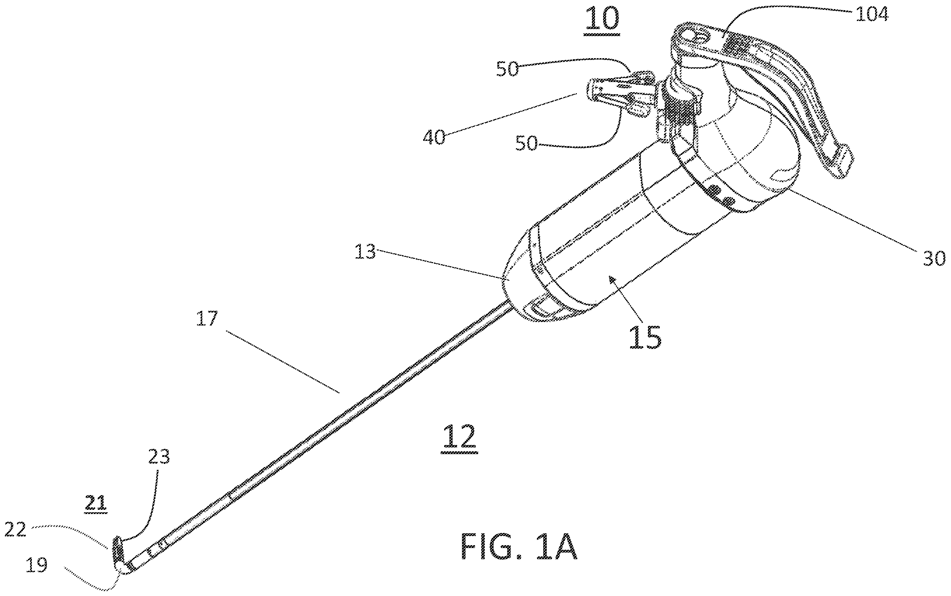

Referring now to the drawings, Figures 1A-12 illustrate an embodiment of the present control unit which is referred to herein as control unit 10.

Figure 1A illustrates control unit 10 having a housing 15 attached to a laparoscopic instrument 12. Laparoscopic instrument 12 includes a proximal housing 13 (releasably attachable to housing 15), shaft 17 having an articulating region 19 and a distal end effector 21 having jaws 22 and 23. Laparoscope 12 is used herein for illustrative purposes, control unit 10 can be attached to, or integrated with, any surgical instrument.

Control unit 10 includes a palm interface 30 which is fixed to housing 15. A drive unit 32 is positioned within housing 15 and controls articulation, effector end operation as well as roll rotation scaling of articulating region 19 of shaft 17. Housing 15 can be fabricated from a polymer and/or alloy using machining, 3D printing and/or casting/molding fabrication approaches. It can be 40-60 mm in diameter and about 60-120 mm in height.

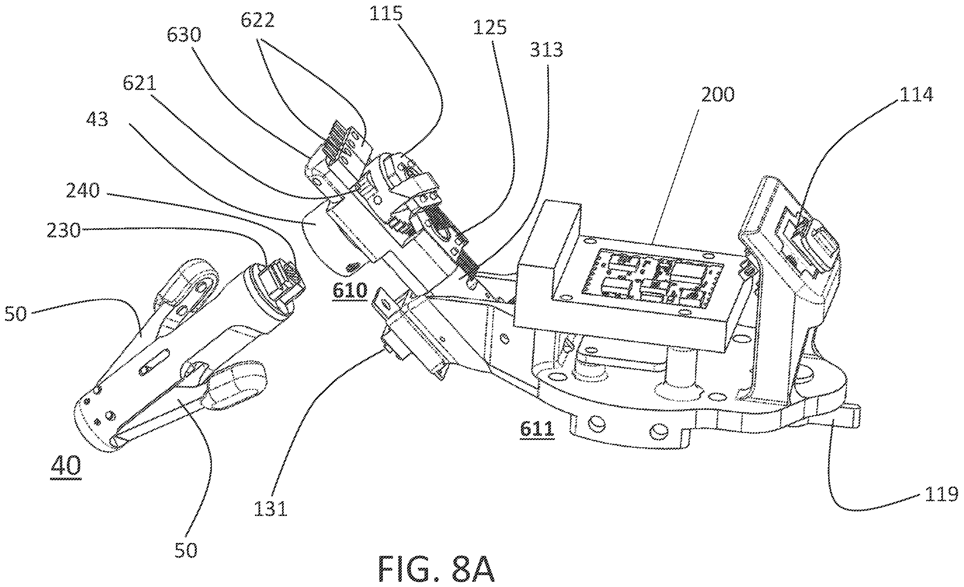

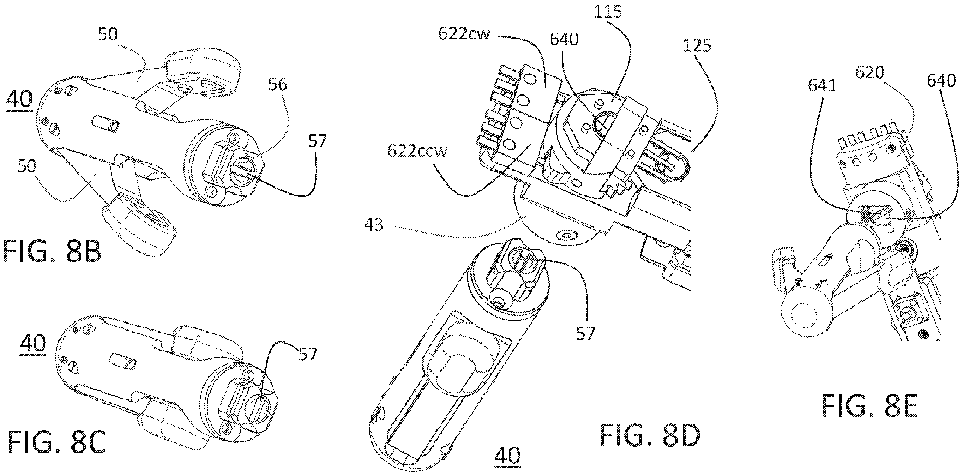

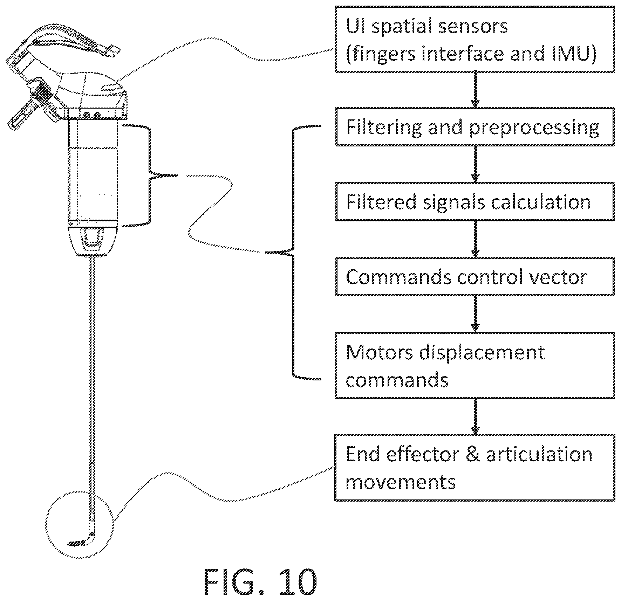

Control unit 10 also includes a finger interface 40 which is connected via hinge to palm interface 30 of control unit 10 (described in greater detail with reference to Figures 8A-F). When finger interface 40 is rotated with respect to palm interface 30 a rotation sensor 125 measures the amount of rotation and generates a signal used to control the deflection of articulating region 19 as is further described hereinbelow with respect to Figure 10.

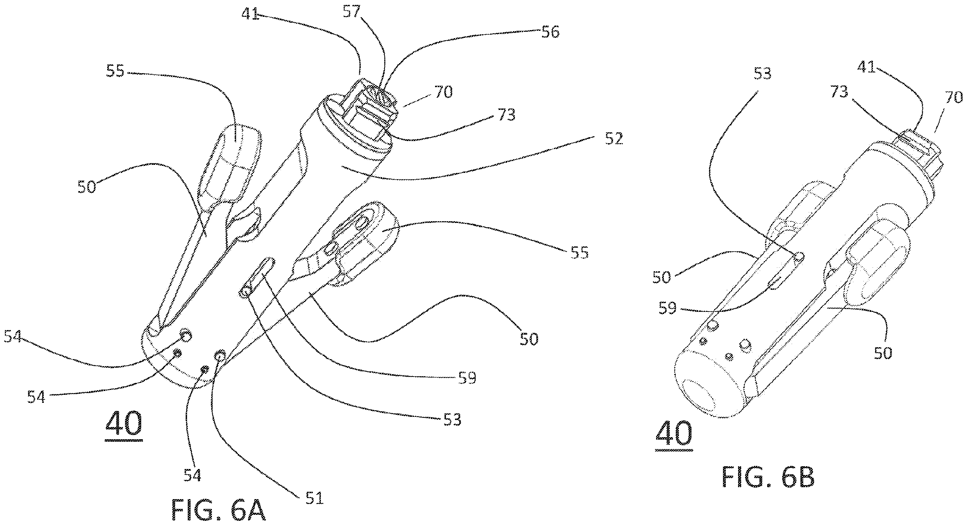

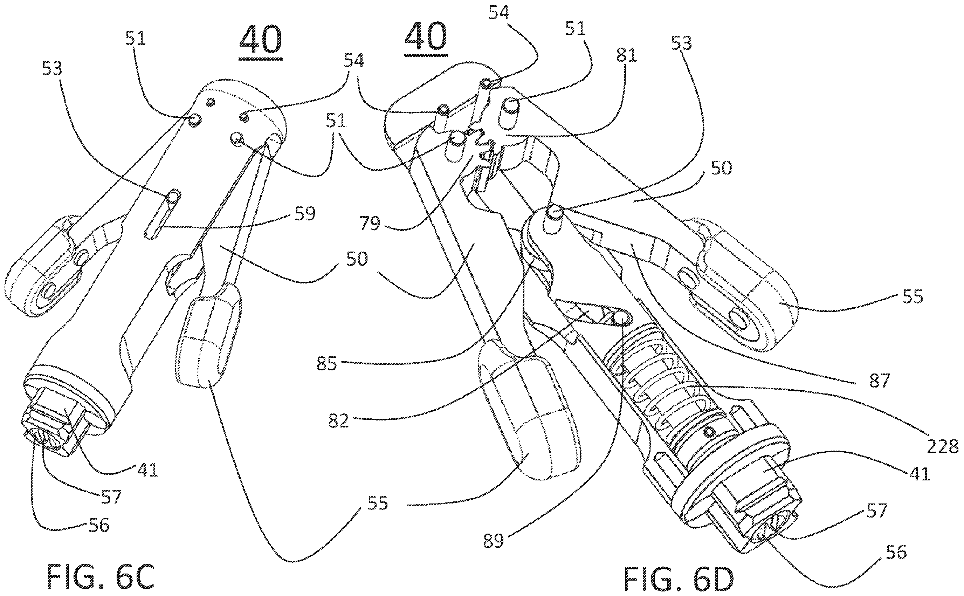

Finger interface 40 of control unit 10 also includes finger levers 50 that can be used to control the movement of the end effector as is further explained hereinbelow with reference to Figures 6A-D and 8A-F.

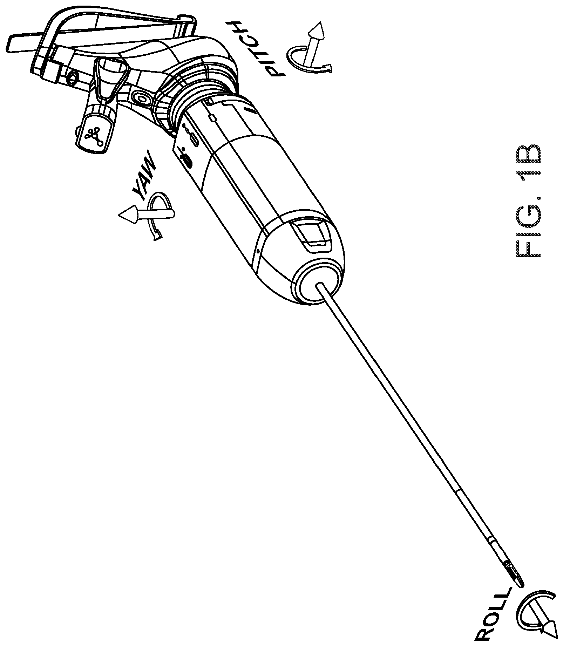

Figures 1B-G illustrate a coordinate systems used to describe the various movements of the control unit and attached device.

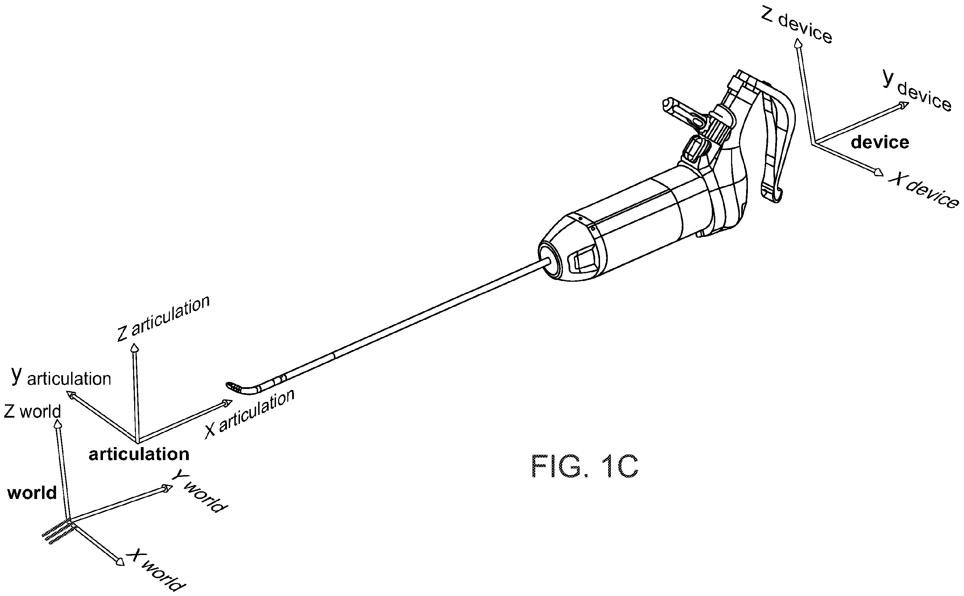

Figure 1B defines pitch, roll and yaw for the control unit. Roll (of the device shaft) is effected by rotating the control unit (left/right) around the long axis of the shaft. Figure 1C illustrates the main coordinate axis as fixed in space. The device coordinate system is fixed to the control unit. As the control unit moves while the surgeon operates, the device coordinate system may be in any position and any orientation with respect to the spatial coordinate system. The Y axis of the device coordinate system is the long axis of the control unit shaft with the X axis of articulation being perpendicular thereto. The articulation coordinate system may rotate around X articulation axis.

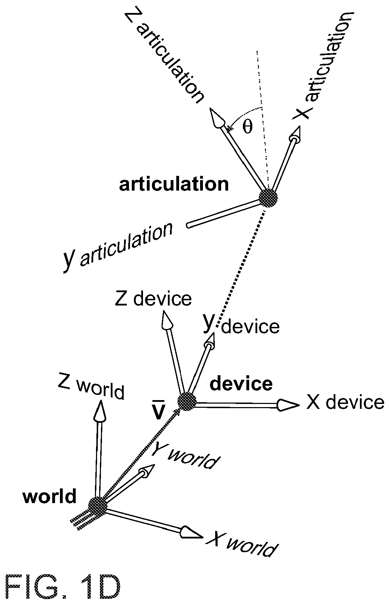

Figure 1D illustrates the relationship between the spatial and device coordinate systems. The origin of the device coordinate system may be positioned in any point and orientation with respect to the world coordinate system. The articulation coordinate system may rotate to any angle Θ.

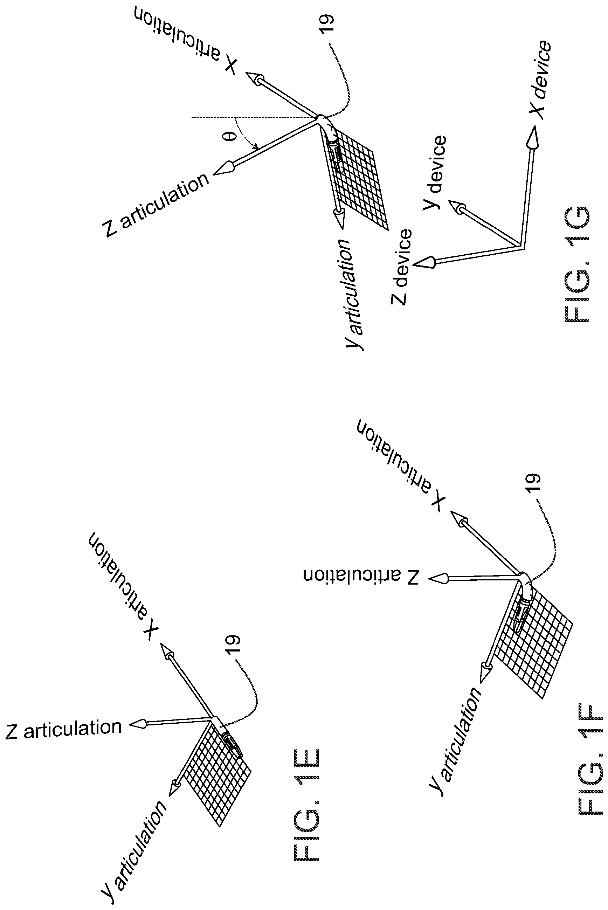

Figures 1E-F illustrate the movement of the articulation with respect to the articulation coordinate system. As shown the articulation bends in the XY plane of the articulation coordinate system. Figure 1E shows the articulation in a straight position and Figure 1F shows the articulation in a bent position. The second movement of the articulation is achieved by rotating the XY articulation plane around the X axis of the articulation coordinate system (Figure 1G). The rotation of the of the XY articulation plane may be achieved by manipulating the articulation cables or by rotating the shaft around its long axis.

In the present invention the bending of the articulation in the XY articulation plane is controlled by tilting the fingers interface and the rotation of the XY of the articulation coordinate system is controlled by rotation sensor fixed to the control unit. The rotation sensors measure control unit rotation and provide a rotation signal to the drive unit in order to rotate the XY plane of the articulation coordinate system. The measured rotation of the control unit is multiplied by a scale factor determined by the surgeon.

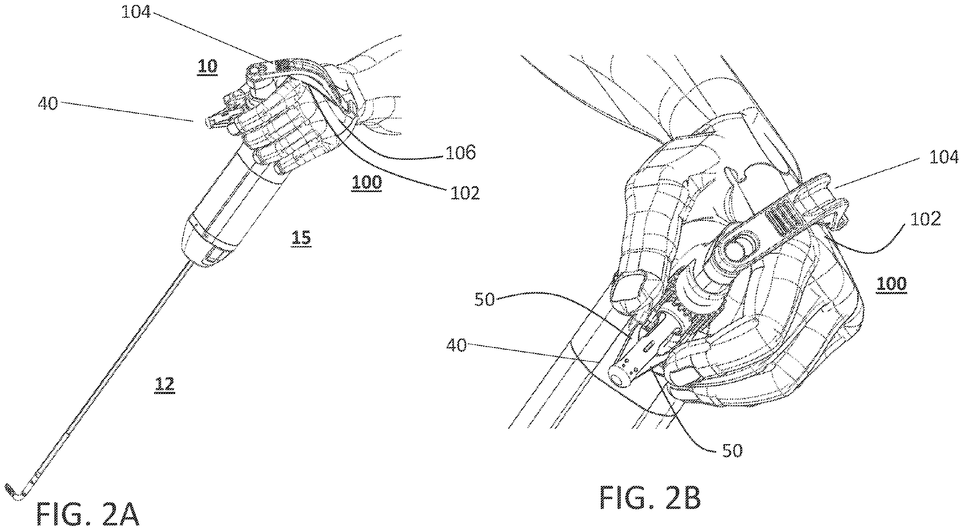

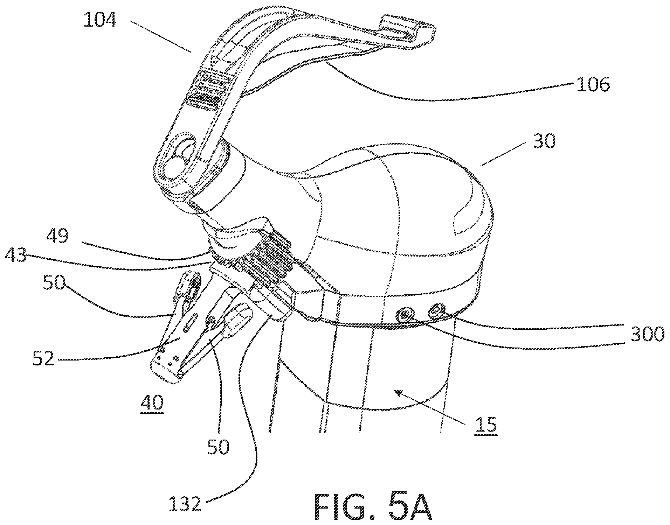

Figures 2A-B illustrate engagement between palm interface 30 and hand 100 of the user. The palm of the user rests against the surface of palm interface 30 with, dorsum 102 of hand 100 positioned underneath dorsum interface 104. Element 106 of dorsum interface 104 is forced downward by elastic deformation (also shown in Figures 5A-G). Three of the user's fingers can be used to grasp the circumference of palm surface 30 while the other two fingers (thumb and index finger) engage (pinch) levers 50 of finger interface 40. While engaging palm interface 30, the user can maneuver (tilt, yaw, up/down) housing 15 and attached laparoscope 12 via housing 13.

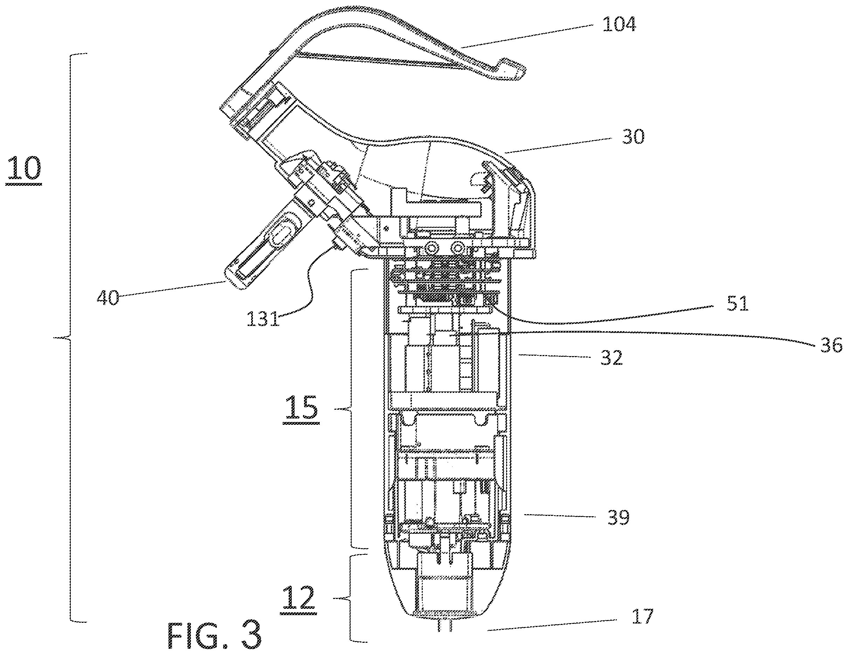

Figure 3 is a cut-away view of control unit 10 showing the main internal components included within housing 15. The interface of control unit 10 forms a part of a proximal portion of housing 15. Housing 15 includes drive unit 32 having at least one motor 36 and related circuitry 34. Housing 15 includes connector 39 for connecting to proximal housing of laparoscope 12.

Sensors located within housing 15 are electrically connected to circuitry 34, allowing the signals from the sensors to be measured and computed in therein. The computed signals are converted by circuitry 34 into action signals that operate motor(s) 36. The rotation of motor(s) 36 are transmitted through connector 37 to the attached laparoscope 12 and converted to movement of the end effector and articulating region 19 of shaft 17.

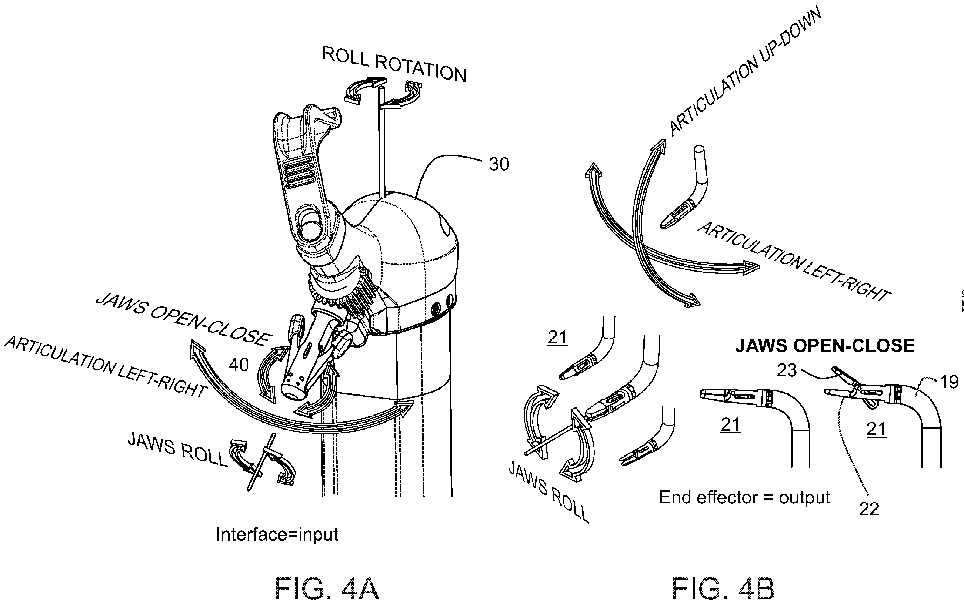

Figures 4A-B illustrate the relationship between movement at interfaces 30 and 40 actuated by the surgeon hand and fingers, and movements of end effector 21 and articulating region 19 as actuated by motor(s) 36. The tilt of finger interface 40 is translated to articulation right-left movement at articulating region 19. The opening and closing of levers 50 of finger interface 40, i.e. changing the distance between the thumb and index fingers, results in open-close of jaws 22 and 23, i.e. reducing the distance between the jaws. Rotating the body of finger interface 40, by rotating the fingers levers, results the roll of the jaws. Roll rotating control unit 10 and attached laparoscope 12 (articulation plane rotation), by rotating palm interface 30 results in roll rotation of the XY plane of the articulation. The latter is by scaling the IMU sensor signals positioned in housing 15 as is further described hereinbelow.

The control of an end effector mechanism i.e. opening and closing the jaws 22, 23 and the rotation of the effector, (shown in Figure 4B), is done by open/close and/or rotate levers 50 of finger interface 40. While manipulating the levers, sensors 115 (open-close) and 125 (rotation) located at housing 15, (Figures 6A-D and 8A-B) are sampled and processed by a controller 34. The processed signals are computed and converted to action signals to motor(s) 36 for actuating the jaw mechanism.

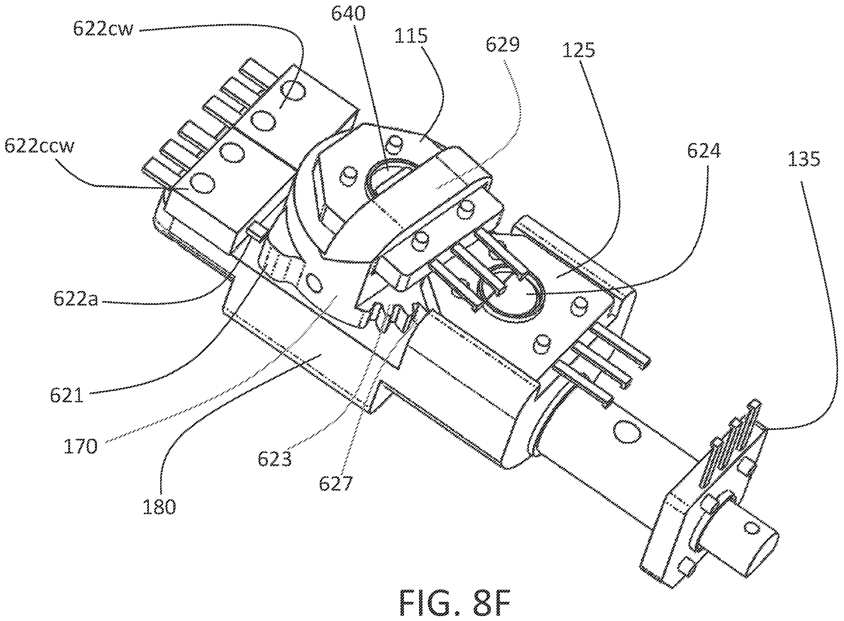

In order to control roll position and deflection of articulating region 19 of shaft 17, controller 34 receives signals from both the IMU-AHRS sensor 200 and tilt sensor 135 shown in Figure 8F. IMU sensor 200 measures the roll rotation of housing 15 about the shaft axis. Tilt sensor (e.g., potentiometer) 135 (Figure 8F) measures the tilt of finger interface 40 with respect to palm interface 30.

The processed signals from the combined IMU-AHRS sensor 200 and tilt sensor 135 are computed and converted to action signals to motor(s) 36 for actuating deflection of articulating region 19.

The IMU portion of IMU-AHRS sensor 200 is an inertial measurement unit capable of detecting linear acceleration using one or more accelerometers and detecting rotational rate using one or more gyroscopes. The IMU can also include a magnetometer which is commonly used as a heading reference. A typical configuration of an IMU can include one accelerometer, one gyro, and one magnetometer per axis for each of the three axes: pitch, roll and yaw.

The AHRS portion of IMU-AHRS sensor 200 is a module capable of providing an attitude of the control unit frame. It can be based on solid-state MEMS sensors block that supplies raw rate gyroscopes, accelerometers and magnetometers readings. These readings are fed into a non-linear statistical filter (such as an extended Kalman Filter) to extract a stable estimate of the attitude information (roll, pitch and yaw). The module can be compact and be integrated in a printed circuit board. In order to align the roll measurement of the AHRS to the shaft axis a calibration needs to take place to account for physical misalignment of the module on the control unit frame. Calibration is effected by placing the control unit in an aligned orientation, such as flat table, and sending an alignment reset command to the AHRS module by the dialog button 131. After the calibration is completed the roll axis measurement is aligned with the device longitudinal axis, the calibration setting are stored in flash memory and may be refreshed when new calibration process is executed. The IMU and the AHRS modules can be integrated in to a single miniature unit, e.g., the MTi^{™} and MTx^{™} units supplied by Xsens Technologies B.V.

The statistical filter applied to the IMU signals produces an optimal solution based on assumptions on the movement itself. While using the control unit the filter results may drift to some extent. If at any time the reaction of articulation to the rolling of the control unit and attached device changes, the surgeon can repeat the calibration by placing the device on a flat table and activating the reset process via predefined clicks of button 131. The drifting phenomena may be reduced by providing additional IMU sensors for example, by mounting at least one more IMU sensor in different orientation than the first IMU within the control unit.

The drift may be further improved by adding assumptions to the Kalman filter regarding device usage. For example, the algorithm can be improved if it takes into account that the device shaft is inserted through an incision (access site) that acts as a pivot. Preliminary data with respect to general kinematics of the device while being used in surgical procedure, such as range of movement, system spatial frequencies, maximal accelerations, etc. can also be taken into account and a bounding restriction on the linear movement can be applied to the filter.

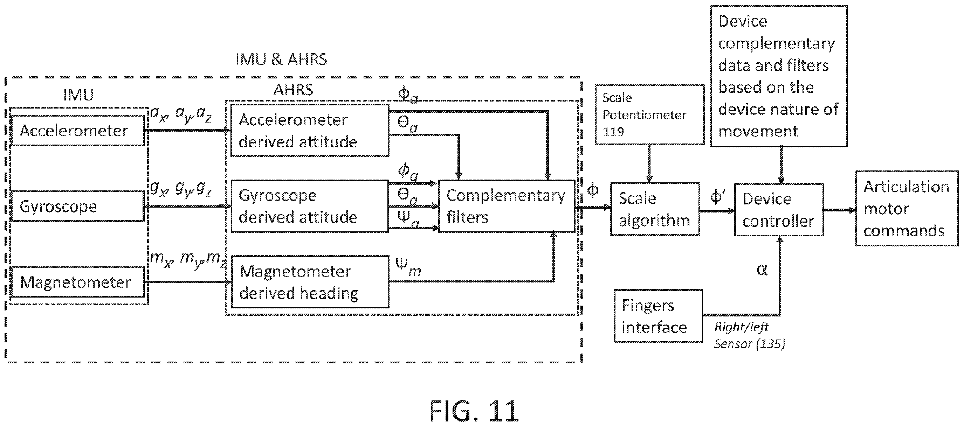

As is shown in Figure 11, the sensing unit provides complete orientation angles for the control unit with respect to a world coordinate system. An AHRS system in that case drives a statistical filter on rate gyroscopes, accelerometers and magnetometers measurements (IMU) to compute a stable estimate of the orientation data (roll, pitch and yaw).

As is mentioned hereinabove, the degree of rotation scaling between the user interface and the articulated region can be selected by the surgeon according to needs. The sum of the mechanical articulation rotation results from the addition of the actual rotation of the control unit body and a scaled rotation resulting from the combined pulling movements of articulation cables.

Scaling can also be used to create an additional scaled tilt quantity on the bending tip plane (the articulation plane). In such a case, the bending tilt can be composed of the signal received from the fingers interface 40 and the control unit rotation as measured by the device controller.

The rotation of the articulation plane, executed by the articulation wires, is effected by the controller. The controller algorithm receives input measurements relating to the device roll relative to the world and the reading from the scaling potentiometer algorithm.

For example, for a 0.0 reading from the scale potentiometer, the bending plane will rotate exactly like the control interface itself. A 30 degree rotation of the control unit will lead to the same angle of rotation of the bending plane. A reading of scale value equal to 2.0 will add 200% to this roll measurement, so for the same control interface rotation of 30 degrees the articulation motors will drive the articulation bending plane for an extra 60 degrees for a total of 90 degree rotation of the bending plane relative to the world coordinate system. Setting a scale value of -1.0 will lead to a fixed bending plane referenced to the world coordinate system meaning that any roll movement of the control interface will not have any effect on the bending plane, i.e. stabilized in space.

Figure 5A, illustrates the main components of the user interface of control unit 10. Palm interface 30 is fixed to housing 15 by screws 300. Dorsum interface 104 is attached to the distal end of palm interface 30. Fingers interface 40 is rotatably attached to the lower side of the neck of palm interface 30.

Fingers interface 40 is connected through coupling 41 to sensor housing 43 (further described with reference to Figures 8A-E). Elastic cover 49 allows coupling 41 to tilt and rotate while protecting the sensor mechanism within housing 43.

The pinch action of the surgeon's fingers is measured through angle of fingers levers 50 with respect to the body 52 of finger interface 40. The lever mechanism converts the angular rotation of the levers to rotation of pin 56 with slot 57 located at the proximal end of pin 56. In this example angular rotation of 25 degrees of the levers is translated to angular rotation of 90 degrees of pin 56, and slot 57.

Figure 8D shows potentiometer 115 with inner shaft 640. Potentiometer 115 measures the rotation of inner shaft 640. As shown in Figure 8E, inner shaft 640 has male bulge 641 that fits slot 57 of fingers interface 40. When fingers interface 40 is attached to housing 43, slot 57 and bulge 641 are coupled and rotate together when levers 50 are moved thus enabling the measurement of a pinch.

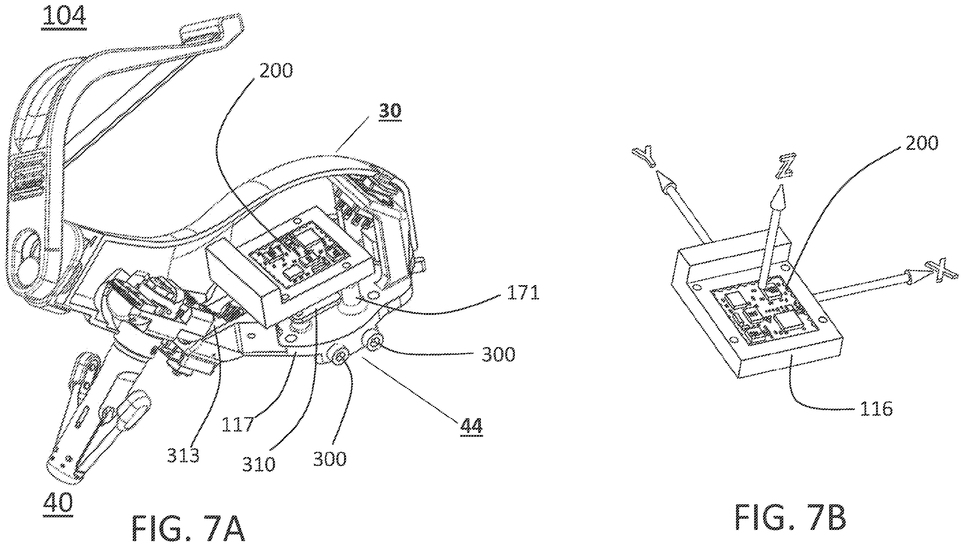

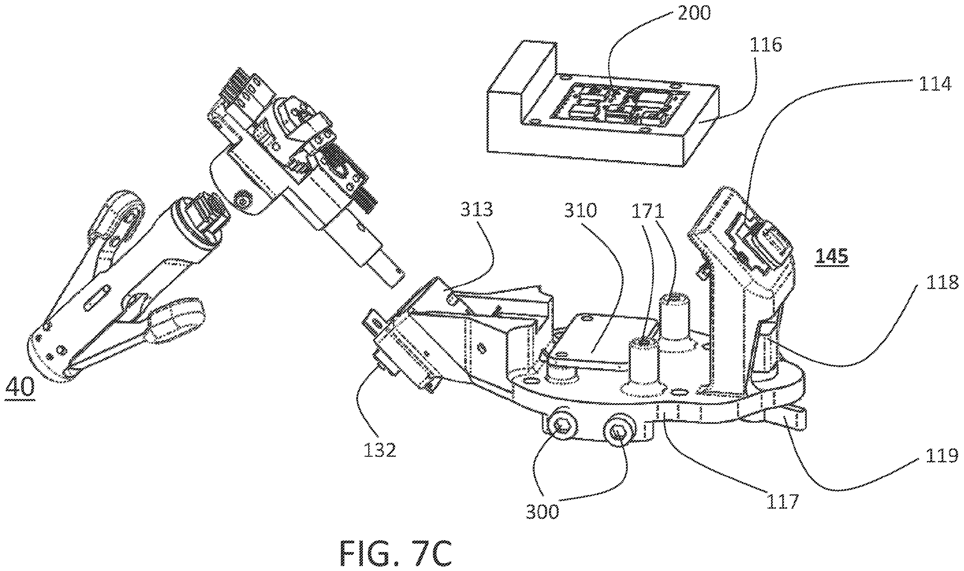

Figure 8F demonstrates the components of the mechanism that measures the rotation of finger interface 40. Base 170 may rotate with respect to body 180 and serves as housing for potentiometer 115 that measures the pinch movement of the fingers at fingers interface 40. Base 170 includes gear 623 which meshes with gear 627 which in turn is connected to shaft 624; the rotational movement of shaft 624 is measured by potentiometer 125. When the fingers interface is rotated, gear 623 rotates causing gear 627 to rotate in the opposite direction. The rotation of gear 627 is measured by potentiometer 125 enabling the measurement of fingers rotation. The controller translate the actual angle of fingers rotation to jaws roll by pre-determined factor chosen by a selected position of switch 114 shown in Figure 7C. Bulge 621 and limit switch sensor 622ccw serves as a mechanical limit to gear 623 rotation. When gear 623 reaches to the rotation limiter further application of torque to levers 50 results in depression of button 622a and a signal from sensor 622ccw commands the motor that rotates the jaws in a selected angular speed in a CCW direction. The same mechanism allows the surgeon to keep the jaws rotating in CW direction by pressing on limit switch 622cw

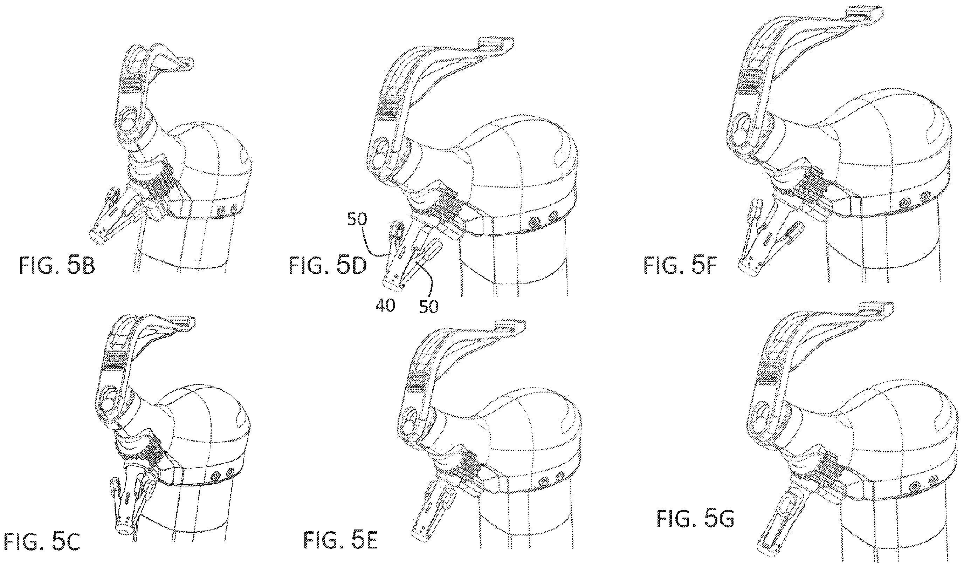

Figures 5B-G illustrate the range of movement in finger interface 40. Figures 5B-C show tilting of finger interface 40 to the right (Figure 5C) and left (Figure 5B). Figure 5D shows levers 50 in an open position while Figure 5E shows levers 50 in a closed (pinched) position. Figure 5F shows Finger interface 40 rotated clockwise (CW), while Figure 5G shows finger interface 40 rotated counter clockwise (CCW).

Figures 6A-D illustrate in detail the structure of finger interface 40. Levers 50 are connected via hinges 51 to cylindrical body 52. Pins 54 are used to limit the rotation of levers 50. Fingers Pads 55 are connected to the distal end of each finger lever 50. When the levers are pressed as is shown in Figure 6B, pin 53 slides within slot 59. As will be further described hereinbelow with reference to Figures 8B-C, the sliding of pin 53 results the rotation of angular sensor 115, connected to pin 56 via slot 57. Pin 53 is located in elongated slot 59 that forces pin 53 to move linearly while levers 50 change their angle with respect to body 52. The linear movement of pin 53 is converted to rotation of pin 56 as is described hereinbelow.

Base 70 is located at the proximal end of body 52. Finger interface 40 can be connected to sensors housing 43 by coupling 41. Groves 73 serve as housing of plungers which are connected at the side of housing 43.

Figure 6D illustrates the inner mechanism of finger interface 40. Levers 50 are connected to body 52 by hinges 51, through gears 79 and 81 of each lever 50. Gears 79 and 81 are meshed which ensures that both levers 50 will travel the same angle. Meshing of these gears also allows the surgeon to operate levers 50 with a single finger if needed. Pins 54 serve as limiters for levers 50 rotation. When levers 50 are pinched, surfaces 85 apply a force on wheel 87 which is rotatably attached to a distal end of pin 56, thus forcing wheel 87 to move linearly toward the proximal base of finger interface 40 against loaded spring 228. Pin 89 is connected to pin 56. Pin 89 is forced to move in spiral slot 82. When pin 53 is forced to move by the forces applied by levers 50 on wheel 83, pin 89 follows the path of spiral slot 82 and causes pin 56 to rotate CW. The rotation of pin 56 is transmitted to sensor 115, via connection of slot 57 to a male connection of the axis that rotates angular sensor 115, allowing the measurement of levers 50 angle. When the surgeon releases the force on levers 50, loaded spring 228 push pin 89 causing pin 89 to rotate CCW. When no force is applied on the levers, the levers reach their maximal angle with respect to body 52, indicating that the jaws of end effector 21 are fully opened.

Figures 7A-C illustrate the structure of the sensors housing 44. The sensors housing 44 is fixed to housing 15 (not shown) and is covered by palm interface 30. The combined IMU and AHRS sensor 200 is connected to plate 116 through columns 171. Electric circuit 310 receives signals from all sensors located in sensors housing 44 and passes them to circuitry 34 in housing 15. The enclosed sensors module includes rotation and open-close sensors of fingers interface 40.

The right-left movement of the fingers is measured via sensor 125 located in housing 313 at the distal end of plate 117.

Module 145 include setup sensors mounted on plate 117. The setup sensors may be used by the surgeon before and during the procedure. Switch 114 can be used to select the range of jaw roll in the end effector. The surgeon may select for example ranges such as ±45 degrees, ±60 degrees or ±90 degrees. Potentiometer may be used to determine scaling between the rotation of housing 15 (and attached laparoscope 12) as measured by IMU sensor and the articulation plane rotation, by moving lever 119 attached to Potentiometer 118, to a desired position. For example scaling up (increasing) the rotation of the articulation plane can be effected by moving lever 119 clock wise and vice versa. A scale factor of 3 for example would result in a plane of the articulation rotation of 3 degrees for every 1 degree of rotation of housing 15 and attached laparoscope 12 (as measured by IMU sensor).

Button 131 is mounted on plate 117 near finger interface 40, and can be used by the surgeon during the procedure to select on the fly desired modes of operation of the attached laparoscope 12.

The control circuits of control unit 10 can be programmed to distinguish between various presses and clicks of button 131 to provide different functionality.

For example, a short press of about 1 second can be used to instruct the jaws of the effector end to stay closed even if the surgeon does not hold finger levers 50 of fingers interface 40. A long press of, for example, 3 seconds can be used to set articulation to a predefined straight position (e.g., 180 degrees).

A single short click can be used to freeze the articulation angle in a desired angle. Double click is used to resume regular mode of operation.

In addition to providing rotation signals for scaling, the combined IMU and AHRS sensor 200 can also be used to provide a surgeon with a hovering mode. This mode can be activated, for example, via 3 short clicks on button 131.

When the hovering mode is activated, control unit 10 maintains end effector 22 at the same spatial orientation regardless of laparoscope 12 positioning.

The control circuits are programed to calculate the spatial orientation of end effector 21 by measuring the spatial position of the device using signals from IMU, and the articulation angle by the known length of the pull cables at the point of activation of the hovering mode.

The controller calculates the changes of the orientation of the device, i.e. the orientation of shaft 17, and activates articulation accordingly in order to maintain the end effector at the same spatial position with respect to a tissue.

This mode can be helpful when the surgeon operates in small constrained body cavities. When using this mode the end effector can "hover" in a desired orientation with respect to a selected organ, allowing the surgeon, for instance, to preform accurate lines of sutures without the need to perform complex maneuvers.

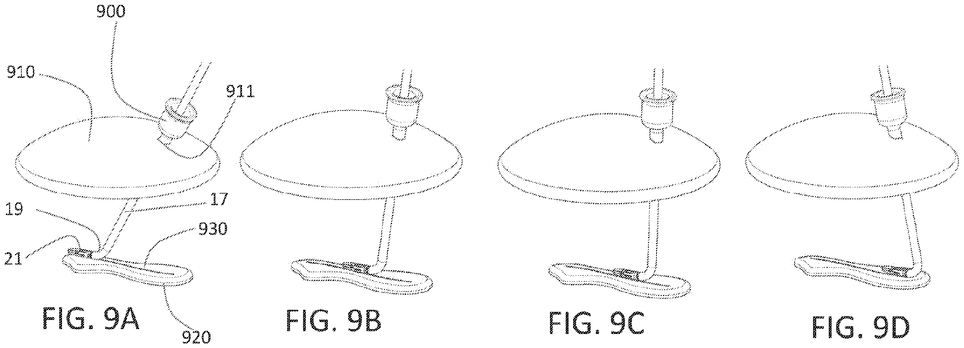

Figure 9A illustrates a surgical procedure for repairing tissue 920 inside the body cavity by suturing cut 930.

In order to begin the suturing procedure aided by the hovering mode, the surgeon positions end effector 21 in the same orientation as cut 930, then the surgeon activates the hovering mode by a sequence of pre-determined clicks on the dialog button 131. From now on, every change in the orientation of shaft 17 will signal motor(s) 36 to correct articulation (via pull wires) in order to maintain the end effector in the same spatial position (articulation correction shown in Figures 9B-D).

Figure 10 illustrates the flow and the processing sequence of signals between the device modules. As is described hereinabove, operating a surgical tool attached to the device, is effected by establishing a functional relationship between finger interface 40 and the orientation of housing 15 containing the combined IMU and AHRS sensor 200, and the action and movement of the end effector (e.g. articulation deflection, jaws open-close and jaws rotation).

As used herein the term "about" refers to ± 10 %.

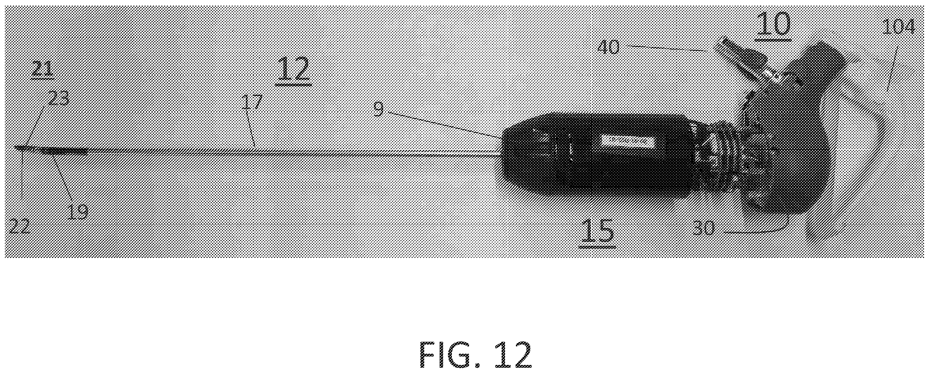

A prototype control unit constructed according to the teachings of the present is shown in Figure 12. The prototype includes a motor pack, control unit rigidly attached to the drive unit and laparoscopic instrument. The motor pack includes four small motors and associated transmissions for actuating the articulation and the end effector in the distal end of the shaft. The size and the weight of the motor pack is small enough to be carried by the surgeon. The interface was fixed to the upper side of the motor pack in the same direction of the shaft axis. The motor pack includes programmable control circuit that allowed the installing control software.

Although the invention has been described in conjunction with specific embodiments thereof, it is evident that many alternatives, modifications and variations will be apparent to those skilled in the art. Accordingly, the invention is defined by the appended claims. In addition, citation or identification of any reference in this application shall not be construed as an admission that such reference is available as prior art to the present invention.

## Claims

1. A control unit (10) for a medical device having an articulating shaft (17) and an end effector (21) the control unit comprising: an interface being engageable by a hand of a user, said interface being immovably attached to a housing of the control unit (10), said housing being attachable to the medical device; and
a sensing unit for measuring a spatial position and orientation of the control unit and the end effector; the control unit (10) being **characterized by** having control circuits programmed to calculate the spatial orientation of the end effector 2. (21) by measuring the spatial position of the device using signals from the sensing unit, and the articulation angle by the known length of pull cables at the point of activation of a hovering mode,
calculate the changes of the orientation of the medical device and
activate articulation accordingly in order to maintain the end effector at the same spatial position with respect to a tissue when the control unit is manoeuvred with respect to said tissue.

2. The control unit (10) of claim 1, wherein said rotational angle of said housing is scaled up or down into said different rotation angle of said articulated region of the shaft.

3. The control unit (10) of claim 1, wherein said interface is a palm interface (30) operable via a palm of said hand.

4. The control unit (10) of claim 1, further comprising a finger interface being pivotally attached to said housing and being engageable by one or more fingers of said hand.

5. The control unit (10) of claim 2, further comprising a restraint being pivotally attached to said palm interface and having an element capable of elastically deforming to apply a restraining force to a back of a hand when said palm is engaged with said palm interface (30).

6. The control unit (10) of claim 4, wherein said finger interface includes levers (50) simultaneously operable via thumb and index finger.

7. The control unit (10) of claim 4, wherein said finger interface is configured for operating articulation of the shaft of the medical device and operating an effector end of the medical device.

8. The control unit (10) of claim 1, further comprising a drive unit (32) in said housing, wherein said drive unit (32) includes at least one motor for controlling the shaft of the medical device.

9. The control unit (10) of claim 8, wherein said sensing unit includes an inertial measurement unit.

10. The control unit (10) of claim 9, wherein a signal from said inertial measurement unit operates said at least one motor to rotate the shaft.

11. A system comprising the control unit (10) of claim 1 attached to a minimally invasive medical device having an articulatable shaft.

## Patentansprüche

1. Steuereinheit (10) für ein medizinisches Gerät mit einem Gelenkschaft (17) und einem Endeffektor (21), wobei die Steuereinheit Folgendes umfasst: eine Schnittstelle, die von einem Benutzer per Hand in Eingriff bringbar ist, wobei die Schnittstelle unbeweglich an einem Gehäuse der Steuereinheit (10) angebracht ist, wobei das Gehäuse am medizinischen Gerät befestigbar ist; und eine Sensoreinheit zum Messen einer räumlichen Position und Ausrichtung der Steuereinheit und des Endeffektors; wobei die Steuereinheit (10) **dadurch gekennzeichnet ist, dass** sie Regelkreise aufweist, die dazu programmiert sind, die räumliche Ausrichtung des Endeffektors (21) durch Messung der räumlichen Position des Geräts unter Verwendung von Signalen von der Sensoreinheit und den Knickwinkel anhand der bekannten Länge von Zugkabeln am Punkt der Aktivierung eines Schwebemodus zu berechnen, die Veränderungen der Ausrichtung des medizinischen Geräts zu berechnen und die Gelenkverbindung dementsprechend zu aktivieren, um den Endeffektor in der gleichen räumlichen Position in Bezug auf ein Gewebe zu halten, wenn die Steuereinheit in Bezug auf das Gewebe manövriert wird.

2. Steuereinheit (10) nach Anspruch 1, wobei der Drehwinkel des Gehäuses auf den anderen Drehwinkel des Gelenkbereichs des Schafts vergrößert oder verkleinert wird.

3. Steuereinheit (10) nach Anspruch 1, wobei die Schnittstelle eine Handflächenschnittstelle (30) ist, die über eine Handfläche der Hand bedienbar ist.

4. Steuereinheit (10) nach Anspruch 1, ferner umfassend eine Fingerschnittstelle, die an dem Gehäuse schwenkbar angebracht ist und durch einen oder mehrere Finger der Hand in Eingriff bringbar ist.

5. Steuereinheit (10) nach Anspruch 2, ferner umfassend eine Rückhaltevorrichtung, die an der Handflächenschnittstelle schwenkbar angebracht ist und ein Element aufweist, das sich elastisch verformen kann, um eine Rückhaltekraft auf einen Handrücken auszuüben, wenn die Handfläche mit der Handflächenschnittstelle (30) in Eingriff steht.

6. Steuereinheit (10) nach Anspruch 4, wobei die Fingerschnittstelle Hebel (50) umfasst, die gleichzeitig über Daumen und Zeigefinger bedienbar sind.

7. Steuereinheit (10) nach Anspruch 4, wobei die Fingerschnittstelle zum Betätigen der Gelenkverbindung des Schafts des medizinischen Geräts und zum Betätigen eines Effektorendes des medizinischen Geräts konfiguriert ist.

8. Steuereinheit (10) nach Anspruch 1, ferner umfassend eine Antriebseinheit (32) in dem Gehäuse, wobei die Antriebseinheit (32) mindestens einen Motor zum Steuern des Schafts des medizinischen Geräts umfasst.

9. Steuereinheit (10) nach Anspruch 8, wobei die Sensoreinheit eine Trägheitsmesseinheit umfasst.

10. Steuereinheit (10) nach Anspruch 9, wobei ein Signal von der Trägheitsmesseinheit den mindestens einen Motor betätigt, um den Schaft zu drehen.

11. System, umfassend die Steuereinheit (10) nach Anspruch 1, die an einem minimalinvasiven medizinischen Gerät angebracht ist, das einen gelenkig verschwenkbaren Schaft aufweist.

## Revendications

1. Unité de commande (10) pour un dispositif médical ayant un axe d'articulation (17) et un effecteur terminal (21), l'unité de commande comprenant :
une interface pouvant être mise en prise par une main d'un utilisateur, ladite interface étant fixée de manière immobile à un boîtier de l'unité de commande (10), ledit boîtier pouvant être fixé au dispositif médical ; et
une unité de détection pour mesurer une position et une orientation spatiales de l'unité de commande et de l'effecteur terminal ;
l'unité de commande (10) étant **caractérisée en ce qu'**elle comporte des circuits de commande programmés pour
calculer l'orientation spatiale de l'effecteur terminal (21) en mesurant la position spatiale du dispositif à l'aide de signaux provenant de l'unité de détection, et l'angle d'articulation par la longueur connue de câbles de traction au niveau du point d'activation d'un mode en suspension,
calculer les changements de l'orientation du dispositif médical, et
activer l'articulation en conséquence afin de maintenir l'effecteur terminal dans la même position spatiale par rapport à un tissu lorsque l'unité de commande est manoeuvrée par rapport audit tissu.

2. Unité de commande (10) selon la revendication 1, dans laquelle ledit angle de rotation dudit boîtier est mis à l'échelle vers le haut ou vers le bas dans ledit angle de rotation différent de ladite région articulée de l'axe.

3. Unité de commande (10) selon la revendication 1, dans laquelle ladite interface est une interface de paume (30) pouvant être actionnée via une paume de ladite main.

4. Unité de commande (10) selon la revendication 1, comprenant en outre une interface de doigt fixée de manière pivotante audit boîtier et pouvant être mise en prise par un ou plusieurs doigts de ladite main.

5. Unité de commande (10) selon la revendication 2, comprenant en outre un dispositif de retenue fixé de manière pivotante à ladite interface de paume et présentant un élément capable de se déformer élastiquement pour appliquer une force de retenue sur le dos d'une main lorsque ladite paume est mise en prise avec ladite interface de paume (30).

6. Unité de commande (10) selon la revendication 4, dans laquelle ladite interface de doigt comprend des leviers (50) pouvant être actionnés simultanément par le pouce et l'index.

7. Unité de commande (10) selon la revendication 4, dans laquelle ladite interface de doigt est configurée pour actionner une articulation de l'axe du dispositif médical et actionner un effecteur terminal du dispositif médical.

8. Unité de commande (10) selon la revendication 1, comprenant en outre une unité d'entraînement (32) dans ledit boîtier, dans laquelle ladite unité d'entraînement (32) comprend au moins un moteur pour commander l'axe du dispositif médical.

9. Unité de commande (10) selon la revendication 8, dans laquelle ladite unité de détection comprend un mesureur inertiel.

10. Unité de commande (10) selon la revendication 9, dans laquelle un signal provenant dudit mesureur inertiel actionne ledit au moins un moteur pour faire tourner l'axe.

11. Système comprenant l'unité de commande (10) selon la revendication 1 fixée à un dispositif médical invasif au minimum ayant un axe articulable.
